# FASCICULE DE BREVET EUROPEEN

(11) **EP 0 577 538 B1**
(45) Date de publication et mention de la délivrance du brevet: **13.08.1997**
(21) Numéro de dépôt: 93480080.6
(22) Date de dépôt: 24.06.1993
(51) Int. Cl.: A61M 5/20

(54) **Dispositif électromécanique d'injection à usage médical et vétérinaire actionné par une gâchette**
Fingerhebelbediente elektromechanische Injektionsvorrichtung für medizinischen und tierärzlichen Gebrauch
Trigger actuated electromechanical injection device for medical or veterinary purposes

(30) Priorité: 01.07.1992 FR 9208339
(43) Date de publication de la demande: 05.01.1994
(73) Titulaire: Denance, Raymond, F-06270 Villeneuve Loubet (FR)
(72) Inventeur: Denance, Raymond, F-06270 Villeneuve Loubet (FR)
(74) Mandataire: Hautier, Jean-Louis

(56) Documents cités:
- FR-A- 2 348 709
- FR-A- 2 390 175
- FR-A- 2 524 321
- US-A- 4 613 328

## Description

L'invention a pour objet un dispositif électromécanique d'injection à usage médical et vétérinaire actionné par une gâchette.

L'état de la technique peut être défini par les brevets suivants :
- FR-A-2.524.321 : L'invention décrit un dispositif d'injection utilisable soit en injection manuelle ou en injection mécanique caractérisé en ce qu'il comporte : une crosse bâtie ayant la forme d'un révolver, une seringue d'injection amovible pourvue d'un piston de seringue, d'une aiguille d'injection unique ou d'un multi-injecteur portant plusieurs aiguilles d'injection, un berceau de seringue rendant solidaire cette seringue à la crosse bâtie, des moyens pour positionner l'impact et prédéterminer le degré de pénétration de l'aiguille ou des aiguilles, des moyens de transmission mécanique comprenant un cliquet et un barillet transmettant la poussée exercée pour déclencher la pénétration de ou des aiguilles et l'injection du liquide.
- FR-A-2.567.760 : L'invention décrit un appareil du type comportant un bâti ayant la forme générale d'un pistolet muni d'une crosse et recevant un chariot mobile pour une seringue amovible à aiguille et piston Il est caractérisé en ce qu'il est équipé d'un seul élément moteur, ici un distributeur à fluide, relié uniquement à un poussoir maintenu en appui contre le piston de la seringue. La liaison cinématique est du type à sens unique. Le chariot est associé à une butée fixe à une distance inférieure à la course totale possible du piston. Ainsi, dans le sens arrière-avant, le poussoir, entraîné, pousse l'ensemble piston-seringue-chariot en appuyant sur le produit placé dans la seringue. Quand le chariot rencontre la butée, il s'arrête et immobilise la seringue, mais le poussoir continue sa course et oblige maintenant le produit à s'écouler par l'aiguille. Dans le sens avant-arrière, une tige solidaire du piston ramène le chariot par une butée qu'elle porte et cela entraîne l'ensemble seringue-piston-poussoir (ce dernier redevenu libre) à revenir en arrière en même temps. Le chariot et la seringue retrouvent leur position de départ tandis que le piston revient un peu moins loin, d'un distance qui est fonction de la dose de produit poussée par lui pendant la course arrière-avant précédente.
- FR-A-2.390.175 : L'invention décrit un dispositif d'injection formant injection automatique. Ce dispositif fonctionne coup par coup ou par rafale, il est logé de préférence dans un bâti et comporte une seringue d'injection de préférence amovible et interchangeable, et des premiers moyens moteurs et des seconds moyens moteurs actionnant celle-ci en déplacement alternatif.
- FR-A-2.594.341 : L'invention décrit un appareil comprenant une tête d'injection porteuse d'une aiguille reliée à une seringue à piston. Un microvérin pneumatique à tige commande le mouvement de l'aiguille pour la piqûre. Un micromoteur réversible commande la vitesse et l'amplitude de déplacement du piston pour régler la quantité et le débit du fluide médicamenteux que la seringue délivre à l'aiguille. Le fonctionnement du micromoteur et du microvérin est sous le contrôle d'une centrale électronique de commande. Il est possible de programmer des injections continues, à retrait temporisé et par salves à cadence variable.
- FR-A-2.622.457 : l'invention décrit un dispositif commandé par microprocesseur pour administrer -ou prélever- des produits liquides ainsi que des micro-courants. Il est constitué par un bâti dont la partie supérieure amovible renferme une seringue munie d'une aiguille. Un micro-cicuit électronique commande le fonctionnement d'un moteur électrique qui actionne une crémaillère assurant la pénétration et le retrait de l'aiguille, et un moteur électrique qui anime une crémaillère actionnant le piston. Des potentiomètres actionnent le piston. Des potentiomètres programment respectivement la puissance et la durée de fonctionnement du moteur, la synchronisation des moteurs, le seuil de sensibilité d'un circuit électronique sensible à des variations de conductibilité au niveau de la pointe de l'aiguille ainsi que 'intensité d'un courant engendré par un second circuit électronique.
- FR-A-2.348.709 : l'invention concerne un dispositif d'injection formant micro-injecteur automatique. Ce dispositif fonctionne coup par coup ou par rafales, il est logé de préférence dans un bâti en forme de pistolet et comporte une seringue d'injection de préférence amovible et interchangeable, et des moyens moteurs, actionnant celle-ci en déplacement alternatif.
- FR-A-2.639.234 : l'invention concerne un dispositif d'injection du type électrique comprenant une crosse bâti, une seringue qui repose sur la face supérieure horizontale de la crosse bâti et est maintenue dans la partie avant par son embout qui repose sur un berceau et dans la partie arrière par la tête du piston de la seringue qui est logée dans un curseur mobile actionné par un moteur électrique qui transmet la poussée pour déclencher la pénétration de l'aiguille et l'injection du liquide, une mire stabilisatrice de la peau guide et prédétermine le degré de pénétration de l'aiguille par un réglage caractérisé par le fait que la mire stabilisatrice est du type amovible, un moyen de maintien faisant office de ressort assure son mientien dans une vis creuse micrométrique, elle-même logée dans un cylindre mobile, ledit cylindre mobile comportant un doigt mobile qui agit sur un moyen faisant office de clapet disposé au niveau de l'aiguille d'injection. La tête du piston de la seringue est en contact avec la tête d'un piston situé dans le curseur mobile, le piston est pourvu d'un ressort monté coaxialement à la tige du piston, ledit ressort est logé dans le curseur mobile, l'ensemble curseur et piston est actionné par le moteur électrique qui agit sur un axe fileté, sur lequel est monté le corps du curseur qui forme à ce niveau un écrou de manoeuvre.

Ces différents dispositifs ou pistolets s'appliquent notamment à la mésothérapie.

Ils permettent des injections continues, cadencées ou répétitives, ou des injections coups par coups.

Le brevet FR-A-2.639.234 décrit un pistolet qui fonctionne sans gâchette, la pénétration de l'aiguille et le départ d'injection se font par simple pression de l'ensemble du pistolet et par rétraction de la mire.

Le brevet US-4.613.328 décrit un appareil d'injection biomédical. Cet appareil utilise un moteur qui actionne le piston de la seringue. Un circuit électronique associé à un cerveau électronique permet de gérer les fonctions de l'appareil d'injection biomédical.

L'inconvénient principal réside dans le fait qu'il faut lever à chaque fois le pistolet, c'est-à-dire quitter le contact au niveau de la mire avec la peau, pour actionner à nouveau le dispositif. Cela peut poser des problèmes dans le cas où l'on traite des surfaces délicates et très précises par exemple sur le visage aussi bien en injection coup par coup, en continu ou injection cadencée ou répétitive.

Pour les autres pistolets qui ont une gâchette, ils utilisent des électroaimants et/ou un mécanisme pneumatique. Ces modes de réalisation sont trop lourds (électro-aimant, bobines, réserves de CO2, etc.) ou bien lorsqu'ils sont ou non manuels, ils sont reliés par des tuyaux à des compresseurs, donc peu pratiques.

L'invention tend à résoudre tous ces inconvénients.

Le dispositif selon l'invention permet trois modes de fonctionnement actionnés par la gâchette, donc sans que la mire soit obligée de quitter le contact avec la peau :
- injection continue,
- injection coup par coup avec des doses préréglées,
- injection cadencée ou répétitive, la cadence de ce va et vient est réglable.

De plus, le dispositif selon l'invention permet de réduire de moitié le poids de l'appareil par rapport aux pistolets équivalents. En effet, le dispositif selon l'invention utilise des moyens électromécaniques tels que des moteurs électriques et des jeux de cames.

Le dispositif électromécanique d'injection à usage médical et vétérinaire selon l'invention telle que décrite dans la revendication 1 est du type comportant un bâti ayant la forme générale d'un pistolet muni d'une crosse et recevant un chariot mobile pour une seringue amovible à aiguille et piston, unmoteur électrique actionnant un poussoir qui agit contre le piston de la seringue et un berceau coulissant support de la seringue monté sur le chariot mobile, actionné lui même par un autre moteur électrique, la partie avant du pistolet est pourvue d'une mire stabilisatrice de la peau qui guide et prédétermine le degré de pénétration de l'aiguille par une molette de réglage qui permet de régler la profondeur d'entrée de l'extrémité de fixation de la mire, un moteur électrique actionne le piston de la seringue est solidaire du chariot mobile, son axe actionne un jeu d'engrenages monté sur ledit chariot mobile caractérisé par le fait que l'autre moteur électrique solidaire du bâti agit, par son axe, sur un jeu de cames qui entraînent les mouvements du chariot mobile. Les mouvements du chariot mobile sont modifiés en actionnant la gâchette du pistolet. La gâchette a son axe parallèle à l'axe du moteur qui entraîne les mouvements du chariot mobile. La gâchette détermine le type d'injection. Par type d'injection, on entend les différents modes d'injection déjà connus et qui sont les suivants : injection continue, injection coup par coup avec doses préréglées, injection cadencée ou répétitive à cadence réglable.

La gâchette du pistolet est montée rotative et déplaçable axialement, elle est pourvue d'une came dont le déplacement en rotation agit sur une autre came montée sur l'axe du moteur d'entraînement du chariot mobile qui est actionné en rotation et en translation et qui, en fonction de la position angulaire de la came de la gâchette, agit pour faire avancer le chariot d'un coup ou en va et vient par des échappements.

La came montée sur l'axe du moteur est une came de transformation du mouvement rotatif en mouvement de translation; à cet effet, elle a une forme de bague pour être emmanchée sur l'axe moteur et comporte au moins une rainure hélicoïdale qui coopère chacune avec un ergot solidaire dudit axe moteur, elle est pourvue de deux oreilles latérales, ladite came fait également office de butée pour venir en butée contre une patte de manoeuvre du chariot mobile.

Le chariot mobile est monté coulissant sur le bâti du pistolet, un ressort de rappel est interposé entre ledit corps du chariot mobile et une paroi du bâti, de manière à renvoyer ledit chariot mobile en arrière.

La gâchette est composée d'une partie libre telle qu'une molette extérieure manoeuvrable en rotation et en translation et d'une seconde partie libre seulement en translation.

Ladite partie libre en rotation et en translation, qui se trouve partiellement à l'intérieur du corps du pistolet, comporte un ergot disposé perpendiculairement à l'axe de la gâchette et qui vient, selon une position angulaire, limiter le déplacement en translation dudit axe de la gâchette.

La seconde partie de l'axe, qui n'est libre qu'en translation, comporte également un ergot qui coopère avec une came en forme de bague pourvue d'au moins une rainure hélicoïdale dans laquelle ledit ergot est disposé.

La came en forme de bague comporte un doigt de positionnement qui, en fonction de l'enfoncement plus ou moins grand de l'axe de la gâchette, va se déplacer de manière uniquement angulaire car elle est bloquée en translation, pour
- soit déplacer la came montée sur l'axe moteur, pour que celle-ci vienne en butée contre la patte de manoeuvre du chariot mobile et le faire avancer pour l'injection en continu au coup par coup,
- soit venir agir contre les deux oreilles latérales pour faire avancer cette came bague pour qu'elle vienne buter contre la patte de manoeuvre et faire avancer le chariot mobile, et ce jusqu'à ce que le doigt de positionnement échappe aux oreilles latérales qui sont en rotation avec la came bague entraînée par l'axe du moteur, pour repartir dans un nouveau cycle dès que le doigt de positionnement rencontre à nouveau les oreilles latérales et ce de manière à obtenir un va et vient du chariot correspondant à l'injection cadencée ou répétitive.

La figure 1 est une vue schématique théorique du dispositif à l'arrêt lorsque la gâchette vient d'être relachée et que le chariot porte-seringue va immédiatement retourner en arrière (voir les flèches F2).

La figure 2 est une vue schématique du dispositif lorsque l'utilsateur appuie sur la gâchette et que celle-ci est enfoncée à fond, le doigt de positionnement est complètement relevé, il vient déplacer, en translation, la came à oreilles montée sur l'axe moteur qui pousse alors la patte de manoeuvre du chariot vers l'avant. Le doigt de positionnement bloque, en retour, l'ensemble de la came sur toute la périphérie circulaire de la came bague. Cette vue correspond à l'injection coup par coup ou en continu.

La figure 3 est une vue du dispositif où la gâchette est enfoncée à mi-course, car elle vient en butée après avoir été actionnée en rotation. Dans cette position, le doigt de positionnement est en contact uniquement avec les oreilles latérales de la came et il échappe très rapidement aux dites oreilles latérales, et par son ressort de rappel, le chariot retourne en arrière. Cette vue correspond à l'injection cadencée ou répétitive.

La figure 4 est une vue selon la figure 3 mettant en évidence l'action du doigt de positionnement et des oreilles latérales, qui déplacent la came en translation, pour pousser la patte de manoeuvre et faire avancer le chariot vers l'avant.

La figure 5 est une vue suivant les figures 3 ou 4 où le chariot est revenu en arrière, après que le doigt de positionnement ait échappé aux oreilles latérales de la came. Les dites oreilles latérales vont venir en butée à nouveau contre le doigt de positionnement et bloquer en rotation et vont à nouveau déplacer la came en translation pour que celle-ci fasse à nouveau avancer le chariot mobile en butant contre la patte de manoeuvre dudit chariot placé sur le chemin de translation de la came montée coulissante sur l'axe moteur.

La figure 6 est une vue en perspective de la came en forme de bague montée sur l'axe moteur, qui est une came de transformation du mouvement rotatif en mouvement angulaire ; elle comporte deux rainures hélicoïdales latérales et deux oreilles latérales.

La figure 7 est une vue de la gâchette avec ses deux parties, la première qui est libre en rotation et en translation, et la seconde qui n'est libre qu'en translation.

La figure 8 est une vue en perspective de la came en forme de bague montée sur la seconde partie de l'axe de la gâchette ; elle est pourvue d'un doigt de positionnement. Ladite came est animée en rotation par deux ergots solidaires de l'axe et deux rainures hélicoïdales latérales, cette came n'est pas libre en translation.

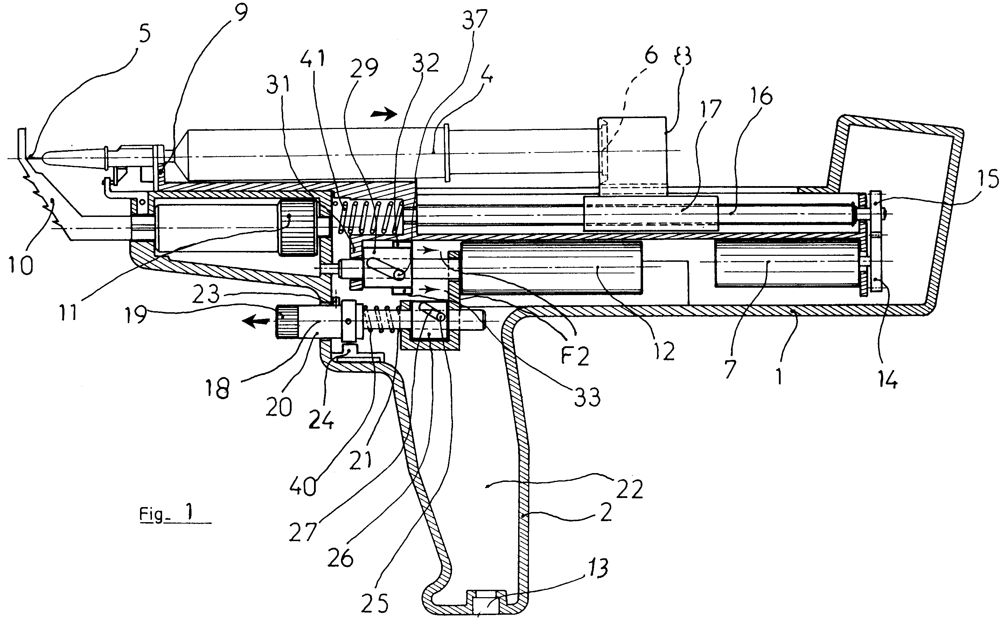

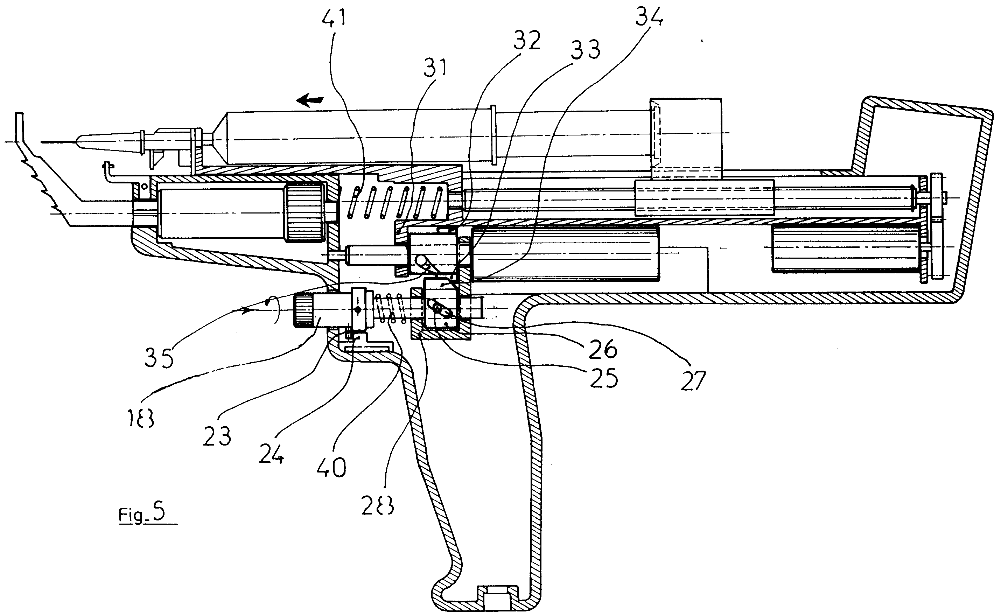

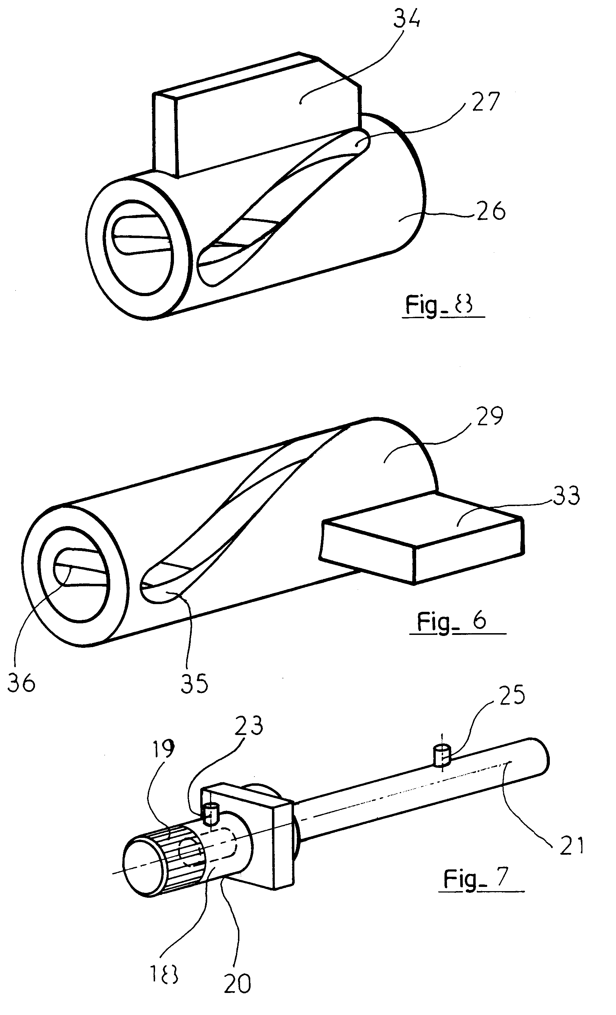

Le dispositif electromécanique d'injection à usage médical et vétérinaire, tel que représenté sur les figures 1 à 7, est du type comportant un bâti 1 ayant la forme générale d'un pistolet muni d'une crosse 2. La crosse comportant la partie électronique n'est pas représentée. Ce bâti 1 reçoit un chariot mobile 3 pour une seringue amovible 4 à aiguille 5 et piston 6, au moins un élément moteur tel qu'un moteur électrique 7 actionne un poussoir 8 qui agit contre le piston 6 de la seringue. La seringue 4 est montée sur un berceau 9 qui fait office de support à ladite seringue 4. Ledit berceau est solidaire du chariot mobile 3. Le poussoir 8 est monté coulissant sur une tige filetée 16 qui est elle-même actionnée par ledit moteur 7.

La partie avant du pistolet est pourvue d'une mire stabilisatrice 10 de la peau qui guide et prédétermine le degré de pénétration de l'aiguille 5 par une mollette 11 de réglage qui permet de régler la profondeur d'entrée de l'extrémité de fixation de la mire 10. Tous ces différents moyens sont connus par l'état de la technique.

Dans le mode de réalisation représenté sur les figures, le moteur 7 est solidaire du chariot mobile 3. Il se déplace avec celui-ci.

Le moteur 7 est alimenté par une batterie ou par un raccordement électrique qui peut s'effectuer par la sortie 13 de la crosse 2 du pistolet.

L'axe du moteur 7 actionne un jeu d'engrenages 14, 15, solidaire également du bâti 3. L'engrenage 15 fait tourner la tige filetée 16, qui déplace le curseur 17, qui est solidaire du poussoir 8 qui va actionner le piston 6 de la seringue 4.

L'autre moteur 12 est solidaire du bâti 1 contrairement au moteur 7 qui est solidaire du chariot mobile 3.

Ce moteur 12 agit, par son axe 30, sur la came 29 à oreilles 32,33 qui entraîne les mouvements du chariot mobile 3. Les mouvements du chariot mobile 3 sont modifiés en actionnant la gâchette 18 du pistolet.

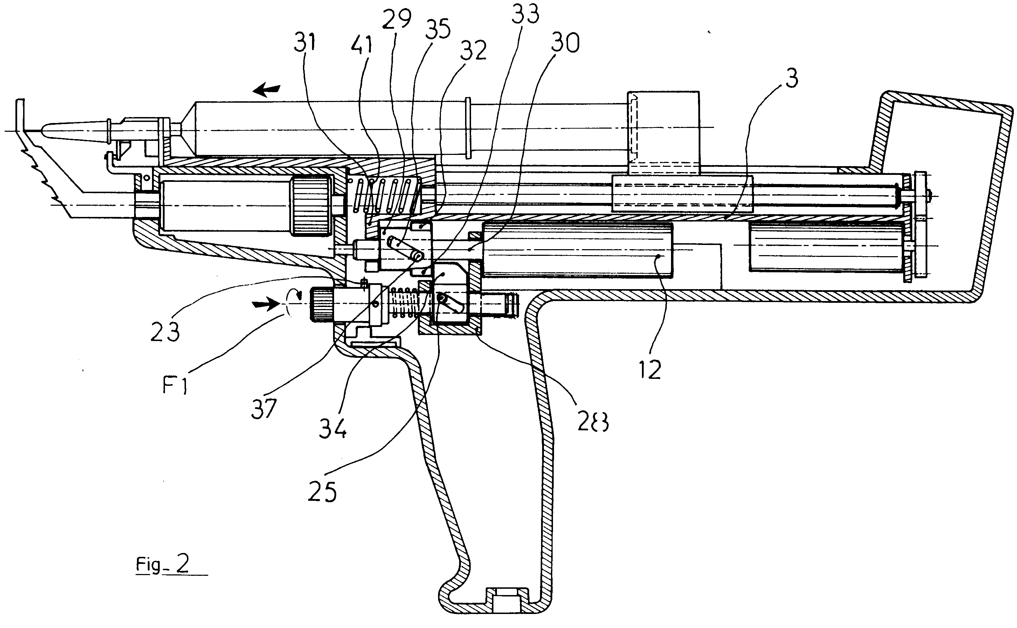

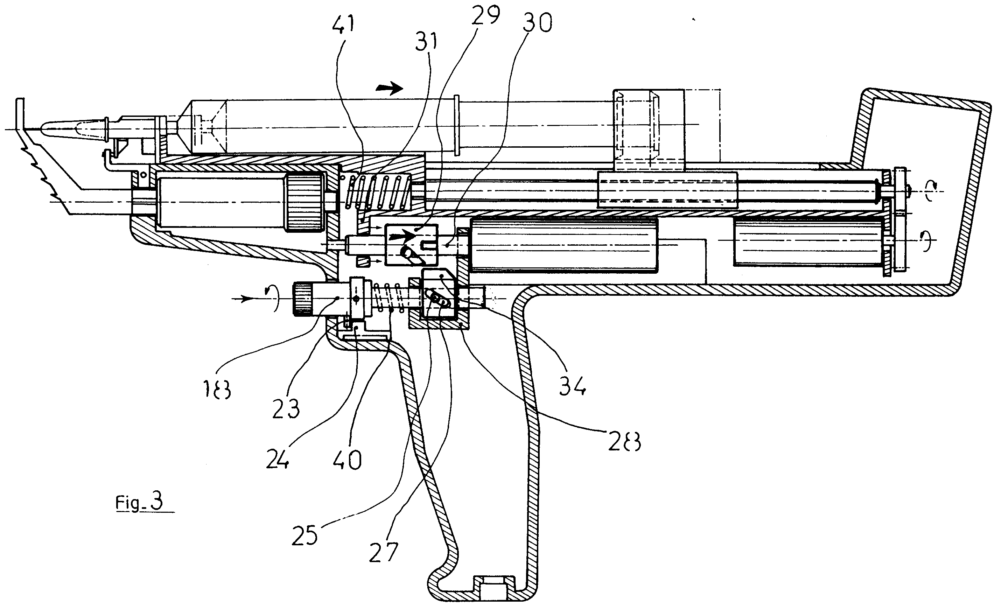

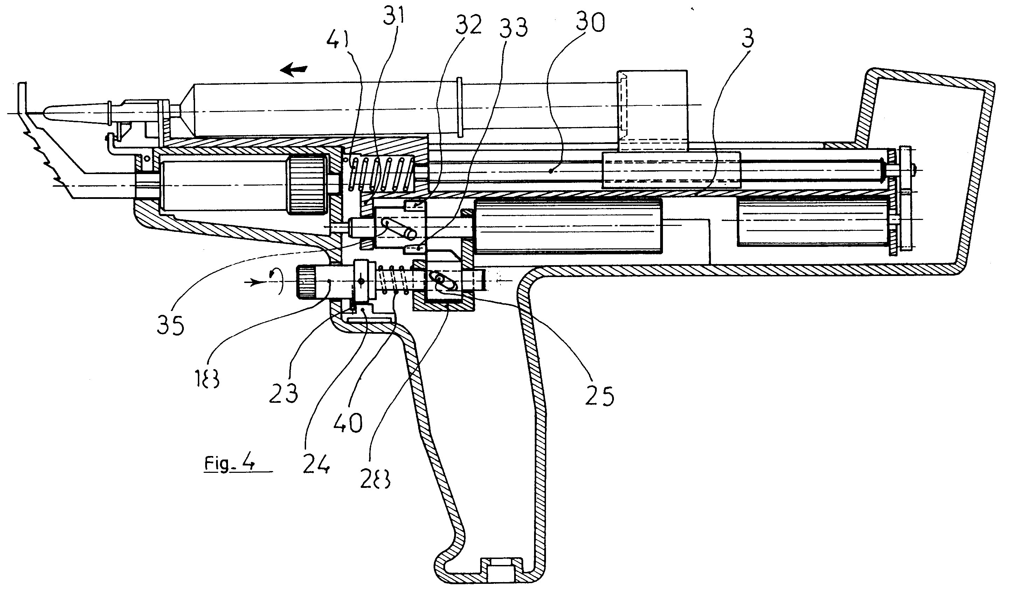

La gâchette 18 est composée d'une partie libre telle qu'une molette extérieure 19 manoeuvrable en rotation, tel qu'indiqué par la flèche F1 (figure 2). Cette première partie 20 est donc libre en rotation et en translation. La seconde partie 21 de la gâchette 18 est libre seulement en translation. La partie 20 libre en rotation et en translation se trouve partiellement dans l'espace intérieur 22 de la crosse du pistolet. Elle comporte un ergot 23 disposé perpendiculairement à l'axe de la gâchette 18. Ledit ergot 23 vient, selon la position angulaire, limiter le déplacement en translation de l'ensemble de la gâchette 18 et ce, du fait que cet ergot 23 peut venir buter contre la butée 24, comme cela est représenté dans les figures 3 et 4. Avec cette position angulaire, la gâchette 18 ne peut être enfoncée que partiellement.

La seconde partie 21 de l'axe de la gâchette, qui n'est libre qu'en translation, comporte deux ergots 25, diamétralement opposés, perpendiculairement à l'axe longitudinal de la gâchette 18. Ces deux ergots 25 coopèrent avec une came 26 en forme de bague pourvue de deux rainures hélicoïdales latérales 27 dans laquelle lesdits ergots 25 sont disposés. La came 26, en forme de bague, comporte, à sa périphérie, un doigt de positionnement longitudinal 34 qui, en fonction de la position en translation de l'axe de la gâchette 18, va se déplacer de manière uniquement angulaire car, ladite came en forme de bague 26 est bloquée, en translation, dans un logement 28 (figure 2). Cette came 26, en forme de bague, avec son doigt de positionnement 34, va, selon l'orientation angulaire du doigt de positionnement :
- soit déplacer la came 29 (figure 2) qui est montée sur l'axe 30 du moteur 12, pour que celle-ci vienne en butée contre la patte de manoeuvre 31 du chariot mobile 3 et le faire avancer pour l'injection en continu ou pour l'injection au coup par coup,
- soit venir agir contre les deux oreilles latérales 32 et 33 pour faire avancer la came 29, pour qu'elle vienne buter contre la patte de manoeuvre 31 et faire avancer le chariot mobile 3, et ce jusqu'à ce que le doigt de positionnement 34 échappe aux deux oreilles latérales 32 et 33 qui sont en rotation avec la came 29 entraînée par l'axe 30 du moteur 12 et ce, pour repartir dans un nouveau cycle dès que le doigt de positionnement 34 rencontre à nouveau les oreilles latérales 32, 33 et ce, de manière à obtenir un va et vient du chariot 3 correspondant à l'injection cadencée ou répétitive.

La came 29 en forme de bague est une came de transformation du mouvement rotatif en mouvement de translation. A cet effet, elle a une forme de bague emmanchée sur l'axe 30, axe du moteur 12. Elle comporte, à cet effet, deux rainures hélicoïdales 35 et 36 qui coopèrent chacune avec des ergots 37 disposés diamétralement opposés perpendiculairement à l'axe 30. Ces ergots sont solidaires de l'axe 30. Selon la position angulaire du doigt de posisionnement 34, celui-ci, par sa hauteur plus ou moins grande, vient, soit en butée contre l'extrémité circulaire de la came 29 en l'ayant fait se déplacer en translation, et celle-ci épouse donc la patte de manoeuvre 31 du chariot mobile 3 soit, lorsque le doigt de positionnement 34 est moins relevé par sa position angulaire, simplement en butée contre les oreilles latérales 32 et 33, provoquant, comme expliqué plus haut, un mouvement de va et vient du chariot mobile 3.

Le mode de fonctionnement du dispositif selon l'invention est le suivant :

### Injection continue :

L'utilisateur doit appuyer sur la gâchette 18. Immédiatement, les moteurs 7 et 12 sont actionnés. Le moteur 7 commande l'injection du produit par translation du piston de la seringue. Le moteur 12 commande la piqûre 5 par translation du chariot mobile 3 portant ladite seringue 4.

Tant que la gâchette 18 est maintenue enfoncée, le chariot mobile 3 reste dans la position avancée et le piston 6 de la seringue 4 avance. En relâchant la gâchette 18, comme représenté à la figure 1, celle-ci revient à sa position initiale par le ressort de rappel 40. La came 29, dégagée du doigt de positionnement 34, revient en arrière, comme indiqué par les flèches F2 représentées à la figure 1. La patte de manoeuvre 31 est libérée, le chariot 3 est ramené en arrière par son ressort de rappel 41, disposé entre une partie du bâti 1 et un épaulement du chariot 3.

### Injection coup par coup

Selon ce mode de fonctionnement, une ou plusieurs doses sont préétablies avec des valeurs déterminées. La dose est programmée par un bouton non représenté. La dose choisie est indiquée par des lampes lumineuses.

Le principe est le même qu'en injection continue mais, par contre, lorsque la dose est administrée, même en laissant la pression sur la gâchette 18, les deux moteurs 7 et 12 sont stoppés. La seringue 4 et le chariot 3 reculent en même temps que le produit n'est plus injecté.

Quand le chariot 3 est revenu, une nouvelle action sur la gâchette 18 permet de recommencer une nouvelle injection coup par coup.

### Injection cadencée ou répétitive

L'utilisateur appuie sur la gâchette 18, les deux moteurs 7 et 12 sont actionnés en même temps. Le dispositif électromécanique de va et vient, tel que décrit plus haut, est appliqué au chariot mobile 3 porte-seringue. La cadence de ce va-et-vient est réglable par un bouton non représenté.

### REFERENCES

- 1.: Bâti du pistolet
- 2.: Crosse du pistolet
- 3.: Chariot mobile
- 4.: Seringue amovible
- 5.: Aiguille
- 6.: Piston
- 7.: Moteur électrique d'injection du produit
- 8.: Poussoir
- 9.: Berceau
- 10.: Mire stabilisatrice
- 11.: Molette
- 12.: Moteur électrique manoeuvrant le chariot mobile
- 13.: Sortie de la crosse pour le raccordement électrique
- 14.: Engrenage
- 15.: Engrenage
- 16.: Tige filetée
- 17.: Curseur
- 18.: Gâchette
- 19.: Molette de la gâchette
- 20.: Première partie de la gâchette
- 21.: Seconde partie de la gâchette
- 22.: Espace intérieur de la crosse du pistolet
- 23.: Ergot
- 24.: Butée
- 25.: Ergot
- 26.: Came
- 27.: Rainures hélicoïdales
- 28.: Logement
- 29.: Came à oreilles
- 30.: Axe du moteur
- 31.: Patte de manoeuvre
- 32.: Oreille latérale de la came
- 33.: Oreille latérale de la came
- 34.: Doigt de positionnement
- 35.: Rainure hélicoïdale
- 36.: Rainure hélicoïdale
- 37.: Ergots
- 40.: Ressort de rappel de la gâchette
- 41.: Ressort de rappel du chariot

## Revendications

1. Dispositif électromécanique d'injection à usage médical et vétérinaire comportant un bâti (1) ayant la forme générale d'un pistolet muni d'une crosse (2) et recevant un chariot mobile (3) pour une seringue amovible (4) à aiguille (5) et piston (6), un élément moteur (7) actionnant un poussoir (8) qui agit contre le piston (6) de la seringue (4) et un berceau (9), support de la seringue (4) monté sur le chariot mobile (3), actionné lui même par un moteur électrique (12), la partie avant du pistolet est pourvue d'une mire stabilisatrice (10) de la peau qui guide et prédétermine le degré de pénétration de l'aiguille (5) par une molette (11) de réglage qui permet de régler la profondeur d'entrée de l'extrémité de fixation de la mire (10), l'élément moteur (7) comprenant un moteur électrique qui actionne le piston (6) de la seringue (4) et est solidaire du chariot mobile (3), l'axe du moteur de l'élément moteur (7) actionne un jeu d'engrenages (14,15) monté sur ledit chariot mobile (3) caractérisé par le fait
que le moteur électrique (12) solidaire du bâti (1) agit, par son axe (30), sur un jeu de cames (26,29) qui entraînent les mouvements du chariot mobile (3) et que les mouvements du chariot mobile (3) sont modifiés en actionnant la gâchette (18) du pistolet ; la gâchette (18) a son axe parallèle à l'axe (30) du moteur (12) qui entraîne les mouvements du chariot mobile (3), la gâchette détermine le type d'injection, notamment injection continue, injection coup par coup avec doses préréglées, injection cadencée ou répétitive à cadence réglable ; la gâchette (18) du pistolet est montée rotative et déplaçable axialement, elle est pourvue d'une came (26) dont le déplacement en rotation agit sur une autre came (29) montée sur l'axe (30) du moteur (12) d'entraînement du chariot mobile (3) qui est actionné en rotation et en translation et qui, en fonction de la position angulaire de la première came (26) de la gâchette (18), agit pour faire avancer le chariot (3) d'un coup ou en va et vient par des échappements ; la came (29) montée sur l'axe (30) du moteur (12) est une came de transformation du mouvement rotatif en mouvement de translation ; à cet effet, elle a une forme de bague (29) pour être emmanchée sur l'axe (30) du moteur (12) et comporte au moins une rainure hélicoïdale (35, 36) qui coopère chacune avec un ergot (37) solidaire dudit axe (30), elle est pourvue de deux oreilles latérales (32, 33), ladite came fait également office de butée pour venir en butée contre une patte de manoeuvre (31) du chariot mobile (3).

2. Dispositif selon la revendication 1, caractérisé par le fait
que le chariot mobile (3) est monté coulissant sur le bâti (1) du pistolet, un ressort de rappel (41) est interposé entre ledit corps du chariot mobile (3) et une paroi du bâti (1), de manière à renvoyer ledit chariot mobile (3) en arrière.

3. Dispositif selon la revendication 1, caractérisé par le fait
que la gâchette (18) est composée d'une première partie libre (20) telle qu'une molette (19) extérieure manoeuvrable en rotation et en translation et d'une seconde partie libre (21) seulement en translation, ladite partie libre (20) en rotation et en translation, qui se trouve partiellement à l'intérieur de la crosse du pistolet, comporte un ergot (23) disposé perpendiculairement à l'axe de la gâchette (18) et qui vient, selon une position angulaire, limiter le déplacement en translation dudit axe de la gâchette (18) ; la seconde partie (21) de l'axe, qui n'est libre qu'en translation, comporte également un ergot (25) qui coopère avec une came (26) en forme de bague pourvue d'au moins une rainure hélicoïdale (35, 36) dans laquelle ledit ergot (25) est disposé.

4. Dispositif selon la revendication 3, caractérisé par le fait
que la came (26) en forme de bague comporte un doigt de positionnement (34) qui, en fonction de l'enfoncement plus ou moins grand de l'axe de la gâchette (18), va se déplacer de manière uniquement angulaire car elle est bloquée en translation, pour :
- soit déplacer la came (29) montée sur l'axe (30) du moteur (12), pour que celle-ci vienne en butée contre la patte de manoeuvre (31) du chariot mobile (3) et le faire avancer pour l'injection en continu ou au coup par coup,
- soit venir agir contre les deux oreilles latérales (32, 33) pour faire avancer cette came (29) pour qu'elle vienne buter contre la patte de manoeuvre (31) et faire avancer le chariot mobile (3), et ce jusqu'à ce que le doigt de positionnement (34) échappe aux oreilles latérales (32, 33) qui sont en rotation avec la came (29) entraînée par l'axe (30) du moteur (12), pour repartir dans un nouveau cycle dès que le doigt de positionnement (34) rencontre à nouveau les oreilles latérales (32,33) et ce de manière à obtenir un va-et-vient du chariot (3) correspondant à l'injection cadencée ou répétitive.

## Claims

1. Electromechanical injection device for medical and veterinary use comprising a casing (1) having the general shape of a gun with a butt (2) and equipped with a mobile carriage (3) to receive a movable syringe (4) with needle (5) and piston (6), a motor element (7) actuating a pusher (8) which acts against the piston (6) of the syringe (4), and a cradle (9) supporting the syringe (4) fitted on the mobile carriage (3) itself actuated by an electric motor (12); the front part of the gun is fitted with a sight (10) for stabilizing the skin and which guides and predetermines the degree of penetration of the needle (5) by an adjusting knurled wheel (11) which allows the depth of penetration of the sight (10) fixing end to be adjusted; the motor element (7) consisting of an electric motor which actuates the piston (6) of the syringe (4) and is an integral part of the mobile carriage (3); the shaft of the motor of the motor element (7) driving a set of gear wheels (14, 15) fitted on the said mobile carriage (3) wherein
the electric motor (12) which forms an integral part of the casing (1) acts through its shaft (30) on a set of cams (26, 29) which drive the movements of the mobile carriage (3) and that the movements of the mobile carriage (3) are modified by actuating the trigger (18) of the gun; the trigger (18) axis is parallel to the shaft (30) of the motor (12) which drives the movements of the mobile carriage (3); the trigger determines the type of injection, notably continuous injection, one-off injection with pre-set doses, series injections or repetitive injections at adjustable rhythms; the trigger (18) of the gun which is fitted to rotate and move axially is fitted with a cam (26) whose movement in rotation acts on another cam (29) fitted on the shaft (30) of the motor (12) driving the mobile carriage (3) which is actuated in rotation and in translation and which, in function of the angular position of the first cam (26) of the trigger (18), acts to move the carriage (3) forward in one movement or in a to-and-fro movement through escapements; the cam (29) fitted on the shaft (30) of the motor (12) is a cam transforming rotary movement into translation movement; to this end, it has the form of a ring (29) to be slid over the shaft (30) of the motor (12) and comprises at least one helicoidal groove (35, 36) which each acts with a pin (37) which is an integral part of the said shaft (30), it is fitted with two lateral lugs ugs (32, 33), the said cam also acting as stop for an operating lug (31) of the mobile carriage (3).

2. Device as per claim 1, wherein
the mobile carriage (3) is fitted to slide on the casing (1) of the gun, a return spring (41) being positioned between the said mobile carriage body (3) and the wall of the casing (1) so as to move back the said mobile carriage (3).

3. Device as par claim 1 wherein
the trigger (18) consists of a first free-moving part (20) such that an outer knurled knob (19) can be operated in rotation and in translation and a second free-moving part (21) only in translation; the said part (20) free-moving in rotation and translation, which is to be found partly inside the gun butt, has a pin (23) arranged perpendicular to the shaft of the trigger (18) and which according to an angular position, limits the translation movement of the said shaft of the trigger (18); the second part (21) of the shaft, which is only free to move in translation, also has a pin (25) which acts with a cam (26) in the form of a ring fitted with at least one helicoidal groove (35, 36) in which the said pin (25) is arranged.

4. Device as per claim 3, wherein
the cam (26) in the form of a ring has a positioning finger (34) which, based on the more or less depression of the shaft of the trigger (18) will move only angularly since it's translation is blocked in order to:
- either move the cam (29) fitted on the shaft (30) of the motor (12) so that this butts against the operating lug (31) of the mobile carriage (3) and moves it forward for continuous injection or one-off injections,
- or acts against the two side lugs (32, 33) in order to move this cam (29) forward so that it butts against the operating lug (31) and moves the mobile carriage (3) forward, and this until the positioning finger (34), escapes from the side lugs (32, 33) which rotate with the cam (29) itself driven by the shaft (30) of the motor (12), to restart a new cycle as soon as the positioning finger (34) once again encounters the side lugs (32, 33) and this so as to obtain a to-and-fro movement of the carriage (3) corresponding to the cadenced or repetitive injection mode.

## Patentansprüche

1. Elektromechanische Injektionsvorrichtung zum humanmedizinischen und veterinärmedizinischen Gebrauch, die ein Gehäuse (1) aufweist, das die allgemeine Form einer Pistole mit einem Griff (2) besitzt und einen beweglichen Schlitten (3) für eine abnehmbare Spritze (4) mit Nadel (5) und Kolben (6) sowie eine Motoreinheit (7) enthält, die ein Druckstück (8) betätigt, das gegen den Kolben (6) der Spritze (4) und einen Spritzenträger (9) wirkt, der die Spritze (4) trägt und auf dem beweglichen Schlitten (3) angebracht ist, der seinerseits von einem Elektromotor (12) angetrieben ist,
wobei der vordere Teil der Pistole mit einem stabilisierenden Aufsetzteil (10) zur Stabilisierung der Haut versehen ist, das zur Führung der Nadel (5) und zur Vorgabe der Einstichtiefe der Nadel (5) über ein Einstellrädchen (11) dient, das die Einstellung der Tiefe der Beabstandung des Auflageendes des Aufsetzteils (10) erlaubt, und die Motoreinheit (7) einen Elektromotor umfaßt, der den Kolben (6) der Spritze (4) betätigt und mit dem beweglichen Schlitten (3) verbunden ist, wobei die Achse des Elektromotors der Motoreinheit (7) ein Zahnradgetriebe (14, 15) antreibt, das am beweglichen Schlitten (3) angebracht ist,
dadurch gekennzeichnet, daß
- der Elektromotor (12), der am Gehäuse (1) befestigt ist, über seine Achse (30) eine Anordnung von Kurvenhülsen (26, 29) betätigt, welche die Bewegungen des beweglichen Schlittens (3) bewirken,
- die Bewegungen des beweglichen Schlittens (3) durch Betätigung des Drückers (18) der Pistole modifizierbar sind,
- die Achse des Drückers (18) parallel zur Achse (30) des Elektromotors (12) verläuft, der die Bewegungen des beweglichen Schlittens (3) bewirkt,
- der Drücker (18) die Art der Injektion bestimmt, insbesondere die kontinuierliche Injektion, die Injektion mit einmaligem Hub und mit vorgegebenen Dosiermengen und die repetierende, sequentielle Injektion, wobei deren Zeittakt einstellbar ist,
- der Drücker (18) der Pistole drehbar und axial verschiebbar angeordnet und mit einer Kurvenhülse (26) versehen ist, deren Verstellung unter Drehung auf eine weitere Kurvenhülse (29) wirkt, die auf der Achse (30) des Elektromotors (12) angeordnet ist, der den beweglichen Schlitten (3) antreibt, die drehbar und längsverschiebbar ist und die je nach der Winkelstellung der ersten Kurvenhülse (26) des Drückers (18) den Schlitten (3) mit einmaligem Hub oder aufgrund von Hemmungen in hin- und hergehender Bewegung betätigt,
- die Kurvenhülse (29), die auf der Achse (30) des Elektromotors (12) angebracht ist, eine Kurvenhülse ist, welche die Drehbewegung in eine Längsbewegung umsetzt,
und
- die Kurvenhülse (29) zu diesem Zweck Ringform besitzt, damit sie auf die Achse (30) des Elektromotors (12) aufschiebbar ist, und mindestens eine Spiralnut (35, 36) aufweist, die jeweils mit einem mit der Achse (30) verbundenen Stift (37) zusammenwirkt, und mit zwei seitlichen Ansätzen (32, 33) versehen ist und ferner auch als Anschlag dient, indem sie an einem Betätigungsansatz (31) des beweglichen Schlittens (3) zum Anschlag kommen kann.

2. Vorrichtung nach Anspruch 1, dadurch gekennzeichnet, daß der bewegliche Schlitten (3) auf dem Gehäuse (1) der Pistole gleitend gelagert ist, wobei eine Spannfeder zwischen dem Körper des beweglichen Schlittens (3) und einer Wand des Gehäuses (1) so angeordnet ist, daß der bewegliche Schlitten (3) nach hinten gedrückt wird.

3. Vorrichtung nach Anspruch 1, dadurch gekennzeichnet, daß
- der Drücker (18) aus einem ersten beweglichen Drückerteil (20), wie beispielsweisee einem außenliegenden Drückerrädchen (19), das drehbar und längsverschiebbar ist, und einem zweiten beweglichen Drückerteil (21) besteht, das lediglich längsverschiebbar ist, wobei das drehbare und längsverschiebbare Drückerteil (20), das sich teilweise im Inneren des Griffs (2) der Pistole befindet, einen Stift (23) aufweist, der senkrecht zur Achse des Drückers (18) angeordnet ist und je nach der Winkelstellung die Längsverschiebung der Achse des Drückers (18) begrenzt,
und
- das zweite Drückerteil (21), das eine Achse darstellt und nur längsverschiebbar ist, ebenfalls einen Stift (25) aufweist, der mit einer ringförmigen Kurvenhülse (26) zusammenwirkt, die mit mindestens einer Spiralnut (35, 36) versehen ist, in welcher der Stift (25) angeordnet ist.

4. Vorrichtung nach Anspruch 3, dadurch gekennzeichnet, daß die ringförmige Kurvenhülse (26) einen Positionierfinger (34) aufweist, der in Abhängigkeit vom mehr oder weniger tiefen Hineindrücken der Achse des Drückers (18) lediglich eine Drehbewegung durchführen kann, da er in Längsrichtung blockiert ist, um
- entweder die Kurvenhülse (29), die auf der Achse (30) des Elektromotors (12) angeordnet ist, zu verschieben, damit sie am Betätigungsansatz (31) des beweglichen Schlittens (3) zum Anschlag kommt und diesen zur Durchführung einer kontinuierlichen Injektion oder zur Durchführung von hubweisen Injektionen vorwärtsbewegt,
- oder auf die beiden seitlichen Ansätze (32, 33) zu wirken, um die zugehörige Kurvenhülse (29) vorwärtszubewegen, damit sie am Betätigungsansatz (31) zum Anschlag kommt und den beweglichen Schlitten (3) vorwärtsbewegt, und zwar, bis der Positionierfinger (34) von den beiden seitlichen Ansätzen (32, 33) freigegeben wird, die sich mit der Kurvenhülse (29) drehen, die von der Achse (30) des Elektromotors (12) mitgenommen wird, um einen neuen Zyklus zu beginnen, sobald der Positionierfinger (34) von neuem auf die seitlichen Ansätze (32, 33) trifft, um so eine hinund hergehende Bewegung des Schlittens (3) zu erzielen, die einer sequentiellen Injektion in zeitlicher Abfolge oder einer repetierenden Injektion entspricht.
